(19) Europäisches Patentamt
European Patent Office
Office européen des brevets

(11) Veröffentlichungsnummer: **0 497 299 A1**

(12) # EUROPÄISCHE PATENTANMELDUNG

(21) Anmeldenummer: **92101442.9**

(22) Anmeldetag: **29.01.92**

(51) Int. Cl.5: **C07D 307/20**, C07D 307/32, C07D 309/30, C07D 309/10, C07D 493/08, C12P 17/04, C12P 17/06, C12P 17/08, A61K 31/335, //(C07D493/08, 321:00,307:00),(C12P17/04, C12R1:80),(C12P17/06, C12R1:80),(C12P17/08, C12R1:80)

Der Anmelder hat eine Erklärung nach Regel 28 (4) EPÜ (Herausgabe einer Probe nur an einen Sachverständigen) eingereicht. Eingangsnummer(n) der Hinterlegung(en): DSM 4209 und DSM 4210.

(30) Priorität: **30.01.91 DE 4102669**

(43) Veröffentlichungstag der Anmeldung: **05.08.92 Patentblatt 92/32**

(84) Benannte Vertragsstaaten: **AT BE CH DE DK ES FR GB GR IT LI LU NL SE**

(71) Anmelder: **HOECHST AKTIENGESELLSCHAFT**
**Postfach 80 03 20**
**W-6230 Frankfurt am Main 80(DE)**

(72) Erfinder: **Zeeck, Axel, Prof. Dr.**
**Brüder-Grimm-Allee 22**
**W-3400 Göttingen(DE)**
Erfinder: **Philipps, Sigrid, Dr.**
**Bultstrasse 12**
**W-3100 Celle(DE)**
Erfinder: **Mayer, Marion**
**Petrikirchstrasse 20**
**W-3400 Göttingen(DE)**
Erfinder: **Göhrt, Axel**
**Arndtstrasse 3**
**W-3400 Göttingen(DE)**
Erfinder: **Granzer, Ernold, Dr. Dr.**
**Falkensteiner Strasse 24**
**W-6233 Kelkheim (Taunus)(DE)**
Erfinder: **Hammann, Peter, Dr.**
**Bürgermeister-Rühl-Strasse 20**
**W-6223 Babenhausen(DE)**
Erfinder: **Kirsch, Reinhard, Dr.**
**Johannesallee 18**
**W-6230 Frankfurt am Main(DE)**
Erfinder: **Thiericke, Ralf, Dr.**
**Auestrasse 35**
**W-6057 Dietzenbach(DE)**

(54) **Decarestrictine und verwandte Verbindungen, Verfahren zu ihrer Herstellung und ihre Verwendung.**

(57) Penicilliumstämme, insbesondere DSM 4209 und DSM 4210, bilden unter spezifischen Fermentationsbedingungen Decarestrictine und Decarestrictinderivate, die antibakterielle und lipidregulatorische Eigenschaften besitzen.

EP 0 497 299 A1

Antibiotika aus Penicillium, wie z.B. das Penicillin, sind schon lange bekannt und werden in großem Maßstab therapeutisch angewandt.

Es wurde gefunden, daß Penicillium-Stämme auch Stoffe ganz anderer Struktur synthetisieren können, nämlich Decarestrictine sowie damit verwandte Verbindungen, welche einen Tetrahydrofuran-Ring oder einen Pyranring als Ringsystem aufweisen. Die Verbindungen besitzen pharmakologische und damit therapeutische Wirksamkeit und insbesondere antibakterielle sowie lipidregulatorische Eigenschaften.

In der Patentanmeldung EP 333 024 sind bevorzugt von Penicillium DSM 4209 und DSM 4210 gebildete Zehnringlactone (Decarestrictine) beschrieben. Bei intensiver Untersuchung dieser Stämme wurde gefunden, daß in Abhängigkeit von den Fermentationsbedingungen überraschenderweise weitere Derivate mit ähnlichen Strukturen gebildet werden, welche deutliche antibakterielle sowie lipidregulatorische Eigenschaften besitzen.

Die Erfindung betrifft somit

1. ein Verfahren zur Herstellung der Verbindungen der Formel I

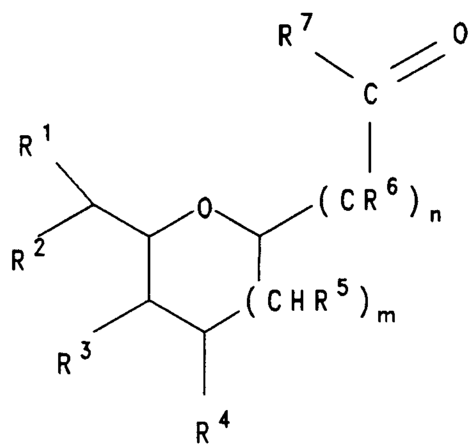

in der

| | |
|---|---|
| $R^1$ | Wasserstoff oder Hydroxyl oder, zusammen mit $R^7$, eine Sauerstoffbrücke bedeutet, |
| $R^2$ | Wasserstoff oder $C_2$ - $C_4$ Hydroxyalkyl ist, |
| $R^3$ und $R^4$ | Wasserstoff oder Hydroxyl bedeutet, wobei mindestens ein Substituent Hydroxyl sein muß, |
| $R^5$ | Wasserstoff oder Hydroxyl ist, |
| $R^6$ | Wasserstoff oder eine Doppelbindung zum Ring-C bedeutet und |
| $R^7$ | Methyl oder Methoxy und unabhängig voneinander m und n = 0 oder 1 sind, |

das dadurch gekennzeichnet ist, daß Penicillium sp., insbesondere DSM 4209 oder DSM 4210, in einem Nährmedium kultiviert wird, bis sich die Verbindung der Formel I in der Kultur anhäuft,

2. eine Verwendung der nach obengenanntem Verfahren hergestellten Verbindungen der Formel I als Pharmazeutika, insbesondere als antibakterielle Mittel sowie zur Regulation des Lipidstoffwechsels.

Im folgenden wird die Erfindung detailliert beschrieben, insbesondere in ihren bevorzugten Ausführungsformen. Ferner wird die Erfindung in den Patentansprüchen definiert.

Die Verbindungen der Formel I werden bevorzugt aus Penicillium sp. DSM 4209 und DSM 4210 isoliert. Diese Stämme wurden aus einer Erdprobe aus dem Bryce Canyon, Utah, USA isoliert und bei der Deutschen Sammlung von Microorganismen nach den Regeln des Budapester Vertrages am 13. August 1987 unter den obengenannten Nummern hinterlegt. Konidien und Sporen des Pilzes wurden wie folgt charakterisiert:

| | |
|---|---|
| Konidien: | Monoverticilliata |
| Sporenoberfläche: | stachelig |
| Sporenfarbe: | graugrün |

In einer Nährlösung, die eine Kohlenstoffquelle und eine Stickstoffquelle sowie die üblichen anorganischen Salze enthält, synthetisiert Penicillium sp., bevorzugt DSM 4209 oder 4210, neben den in EP 333 024 genannten Verbindungen verschiedene Derivate der Formel I. Anstelle der Stämme DSM 4209 oder

4210 können natürlich auch deren Mutanten und Varianten eingesetzt werden, soweit sie Derivate der Formel I synthetisieren. Derartige Mutanten können in prinzipiell bekannter Weise durch physikalische Mittel, beispielsweise Bestrahlung, wie mit UV- oder Röntgenstrahlen, oder durch chemische Mutagene, wie beispielsweise Ethylmethansulfonat (EMS), 2-Hydroxy-4-methoxy-benzophenon (MOB) oder N-Methyl-N'-nitro-N-nitrosoguanidin (MNNG) erzeugt werden.

Als bevorzugte Kohlenstoffquellen für die aerobe Fermentation eignen sich assimilierbare Kohlenhydrate und Zuckeralkohole, wie Glukose, Laktose oder D-Mannit sowie kohlenhydrathaltige Naturprodukte, wie z.B. Malzextrakt. Als stickstoffhaltige Nährstoffe kommen in Betracht:
Aminosäuren, Peptide und Proteine sowie deren Abbauprodukte, wie Peptone und Tryptone, ferner Fleischextrakte, gemahlene Samen, beispielsweise von Mais, Weizen, Bohnen, Soja oder der Baumwollpflanze, Destillationsrückstände der Alkoholherstellung, Fleischmehle oder Hefeextrakte, aber auch Ammoniumsalze und Nitrate. An anderen organischen Salzen kann die Nährlösung beispielsweise Chloride, Carbonate, Sulfate oder Phosphate der Alkali- oder Erdalkalimetalle, Eisen, Zink und Mangan enthalten.

Die Bildung der Verbindungen der Formel I verläuft besonders gut in einer Nährlösung, die etwa 0,2 bis 5 %, bevorzugt 1 bis 4 %, Malzextrakt, 0,02 bis 0,5 %, bevorzugt 0,1 bis 0,4 % Hefeextrakt, 0,1 bis 5 %, bevorzugt 0,5 bis 2 % Glucose und 0,005 bis 0,2 %, bevorzugt 0,01 bis 0,1 % Ammoniumsalze enthält, jeweils bezogen auf das Gewicht der gesamten Nährlösung. Die Kultivierung erfolgt aerob, also beispielsweise submers unter Schütteln oder Rühren in Schüttelkolben oder Fermentern, gegebenenfalls unter Einführen von Luft oder Sauerstoff. Sie kann in einem Temperaturbereich von etwa 18 bis 35 ° C, vorzugsweise bei etwa 25 bis 30 ° C, insbesondere bei 27 bis 28 ° C durchgeführt werden. Der pH-Bereich sollte zwischen 1 und 8 liegen, vorteilhaft zwischen 1 und 4. Man kultiviert den Mikroorganismus unter diesen Bedingungen im allgemeinen über einen Zeitraum von 60 bis 170 Stunden, bevorzugt 100 bis 150 Stunden.

Vorteilhaft kultiviert man in mehreren Stufen, d.h. man stellt zunächst eine oder mehrere Vorkulturen in einem flüssigen Nährmedium her, die dann in das eigentliche Produktionsmedium, die Hauptkultur, vorzugsweise im Volumenverhältnis 1:10, überimpft werden. Die Vorkultur erhält man z.B., indem man ein versportes Mycel in eine Nährlösung überimpft und etwa 48 bis 72 Stunden wachsen läßt. Das versporte Mycel entsteht, wenn man den Stamm etwa 7 Tage auf einem festen oder flüssigen Nährboden, vorzugsweise in Hefe-Malz-Agar, wachsen läßt.

Der Fermentationsverlauf kann anhand des pH-Wertes der Kultur oder des Mycelvolumens durch Dünnschichtchromatographie oder Ausprüfen der biologischen Aktivität überwacht werden. Die Verbindungen der Formel I sind sowohl im Mycel als auch im Kulturfiltrat enthalten.

Die Isolierung der genannten Verbindungen aus dem Kulturmedium erfolgt nach bekannten Methoden unter Berücksichtigung der chemischen, physikalischen und biologischen Eigenschaften der Produkte. Zum Testen der Antibiotika-Konzentration im Kulturmedium oder in den einzelnen Isolierungsstufen kann die Dünnschichtchromatographie, vorzugsweise auf Kieselgel mit Chloroform/Methanol als Laufmittel, verwendet werden, wobei die Menge der gebildeten antibakteriellen Substanz zweckmäßig mit einer Eichlösung verglichen wird.

Zur Isolierung der Verbindungen werden Kulturbrühe und Mycel zuerst mit organischen Lösungsmitteln, vorzugsweise Chloroform oder Essigester extrahiert, um die unpolaren Verunreinigungen zu entfernen. Anschließend wird mit einem stärker polaren Lösungsmittel, vorzugsweise niederen Alkanolen oder Gemischen aus Chloroform und/oder Essigester mit einem niederen Alkanol, extrahiert.

Die Reinisolierung erfolgt vorzugsweise an geeigneten Medien, vorzugsweise an Kieselgel, Aluminiumoxid oder an Ionenaustauschern, durch anschließende Elution mit organischen, polaren Lösungsmitteln oder Lösungsmittelgemischen, wie z.B. Essigester Gemischen aus Essigester und einem niederen Alkanol, gegebenenfalls mit Wasser, bzw. einem für Ionenaustauscher geeigneten Salzgradienten, wie beispielsweise Kochsalz oder Tris-(hydroxymethyl)aminomethan-HCl (Tris-Puffer), und Sammeln der wirksamen Fraktionen.

Die Verbindungen sind in festem Zustand und in Lösungen im pH-Bereich 2 bis 8, insbesondere 3 bis 7, stabil und lassen sich damit in übliche galenische Zubereitungen einarbeiten.

In den folgenden Beispielen wird die Erfindung in weiteren Details beschrieben. Prozentangaben beziehen sich auf das Gewicht, wenn nicht anders angegeben.

Beispiele:

1.

a) Herstellung einer Sporensuspension des Produzentenstammes:

100 ml Nährlösung (2 g Hefeextrakt; 20 g Malzextrakt, 10 g Glucose, 0,5 g $(NH_4)_2PO_4$, 1 l

Leitungswasser, pH-Wert vor dem Sterilisieren 7,3) in einem 500 ml Erlenmeyerkolben werden mit dem Stamm DSM 4209 oder DSM 4210 beimpft und 72 Stunden bei 25 °C und 120 UpM auf der rotierenden Schüttelmaschine inkubiert. Anschließend werden 20 ml Kulturflüssigkeit in einem 500 ml Erlenmeyerkolben mit dem Nährboden der obengenannten Zusammensetzung, dem 20 g Agar/l zur Verfestigung zugegeben wurde, gleichmäßig verteilt und dekantiert. Die Kulturen werden 10 bis 14 Tage bei 25 °C inkubiert. Die nach dieser Zeit entstandenen Sporen eines Kolbens werden mit 500 ml entionisiertem Wasser, das einen Tropfen eines handelsüblichen nichtionischen Tensids (Triton X 100, Fa. Serva) enthält, abgeschwemmt, sofort weiterverwendet oder bei -22 °C aufbewahrt.

b) Herstellung einer Kultur bzw. Vorkultur des Produzentenstammes im Erlenmeyerkolben.

Ein 500 ml Erlenmeyerkolben mit 100 ml der unter a) beschriebenen Nährlösung wird mit einer auf einem Schrägröhrchen gezogenen Kultur oder mit 0,2 ml Sporensuspension angeimpft und auf einer Schüttelmaschine bei 120 UpM und 25 °C inkubiert. Die maximale Produktion der Verbindungen der Formel I ist nach ca. 120 Stunden erreicht. Zum Animpfen von 10 und 100 l Fermentern genügt eine 48 Stunden alte Submerskultur (5 %) aus der gleichen Nährlösung.

2. Herstellung der Verbindungen der Formel I

Ein 10 l Fermenter wird unter folgenden Bedingungen betrieben:

| | |
|---|---|
| Nährmedium: | 20,0 g/l Malzextrakt<br>2,0 g/l Hefeextrakt<br>10,0 g/l Glucose<br>0,5 g/l $(NH_4)_2PO_4$ |
| Inkubationszeit:<br>Inkubationstemperatur:<br>Rührgeschwindigkeit:<br>Belüftung: | 150 Stunden<br>25 °C<br>250 UpM<br>4 l Luft/min. |

Durch wiederholte Zugabe weniger Tropfen ethanolischer Polyollösung kann die Schaumentwicklung unterdrückt werden. Das Produktionsmaximum wird nach ca. 150 Stunden erreicht. Die Ausbeuten liegen zwischen 10 und 100 mg/l.

3. Isolierung der Verbindungen der Formel I

Die Isolierung der erfindungsgemäßen Verbindungen des Typs I erfolgt nach Abtrennung der Biomasse aus dem Kulturfiltrat. Die Abtrennung wird beispielsweise durch Filtration oder Zentrifugation durchgeführt. Das Kulturfiltrat wird über ein Adsorberharz, z.B. auf Polystyrolbasis, gegeben.

Die Elution erfolgt mit einem polaren Lösungsmittel, bevorzugt eingesetzt werden niedere Alkohole, wie z.B. Methanol, die möglicherweise noch mit Wasser vermischt werden.

Die Verbindungen der Formel I können durch Chromatographie an Kieselgel mit Laufmittelsystemen, die Mischungen aus niederen Alkoholen, wie z.B. Methanol, Ethylacetat, n-Alkanen, wie z.B. Hexan oder Heptan, und chlorierten Kohlenwasserstoffen, wie z.B. Methylenchlorid, enthalten, isoliert werden. Auch reine Lösungsmittel sind verwendbar. Nachreinigungen können z.B. mittels HPLC an Kieselgel, Reversed Phase-Kieselgelen oder durch Gelpermeationschromatographie, wie z.B. an Sephadex-LH-20 in Laufmittelsystemen, die Mischungen aus niederen Alkoholen, wie z.B. Methanol, Ethylacetat, n-Alkanen, wie z.B. Hexan oder Heptan, und/oder chlorierten Kohlenwasserstoffen, wie z.B. Methylenchlorid, enthalten, oder auch in reinen Lösemitteln, durchgeführt werden. Verbindungen des Typs I sind entweder viskose Flüssigkeiten oder farblose Feststoffe, welche mit Anisaldehyd-Schwefelsäure nach Erhitzen Farbreaktionen eingehen, wobei die Farbe von Dauer und Stärke des Erhitzens abhängt. Im allgemeinen erhält man bläulich bis grüne Farbreaktionen.

Die Verbindung der allgemeinen Formel I kann zur Prophylaxe und Behandlung von Krankheiten verwendet werden, die auf einem erhöhten Lipidstoffwechsel beruhen oder als antibakterielles Mittel. Die Anwendung betrifft auch pharmazeutische Zubereitungen der Verbindung der Formel I.

Bei der Herstellung von Arzneimitteln können neben dem Wirkstoff auch pharmazeutisch unbedenkliche Zusatzstoffe, wie Verdünnungsmittel und/oder Trägermaterialien, verwendet werden. Hierunter sind physiologisch unbedenkliche Substanzen zu verstehen, die nach Vermischen mit dem Wirkstoff diesen in eine für die Verabreichung geeignete Form bringen.

Geeignete feste oder flüssige galenische Zubereitungsformen sind beispielsweise Tabletten, Dragees, Pulver, Kapseln, Suppositorien, Sirupe, Emulsionen, Suspensionen, Tropfen oder injizierbare Lösungen sowie Präparate mit protrahierter Wirkstoff-Freigabe. Als häufig verwendete Trägerstoffe bzw. Verdünnungsmittel seien z.B. verschiedene Zucker oder Stärkearten, Cellulosederivate, Magnesiumcarbonat, Gelatine,

tierische und pflanzliche Öle, Polyethylenglykole, Wasser oder andere geeignete Lösungsmittel sowie wasserhaltige Puffermittel, die durch Zusatz von Glucose oder Salzen isotonisch gemacht werden können genannt.

Außerdem können gegebenenfalls oberflächenaktive Mittel, Farb- und Geschmacksstoffe, Stabilisatoren, sowie Konservierungsmittel als weitere Zusatzstoffe in den erfindungsgemäßen Arzneimittelzubereitungen Verwendung finden. Es können auch pharmakologisch akzeptable polymere Träger, wie z.B. Polyphenylpyrrolidone, oder andere pharmazeutisch akzeptable Additive, wie z.B. Cyclodextrin oder Polysaccharide, verwendet werden. Die Verbindungen können insbesondere auch mit Additiven, die Gallensäure binden, im Besonderen nicht toxischen, im Gastrointestinal-Trakt nicht resorbierbaren, basischen Anionen-Austauschern, kombiniert werden.

Die Präparate können oral, rektal oder parenteral verabreicht werden. Vorzugsweise können Präparate in Dosierungseinheiten hergestellt werden; insbesondere Tabletten, Kapseln, Suppositorien, stellen BEispiele für geeignete Dosierungseinheiten dar. Jede Dosierungseinheit, insbesondere für die orale Applikation, kann bis zu 1000 mg, bevorzugt jedoch 10 bis 100 mg, des aktiven Bestandteiles enthalten. Jedoch können auch darüber oder darunterliegende Dosierungseinheiten verwendet werden, die gegebenenfalls vor Verabreichung zu teilen bzw. zu Vervielfachen sind.

Gegebenenfalls können die Dosierungseinheiten für die orale Verabreichung mikroverkapselt werden, um die Abgabe zu verzögern, oder über einen längeren Zeitraum auszudehnen,w ie beispielsweise durch Überziehen oder Einbetten des Wirkstoffs in Teilchenform in geeignete Polymere, Wachse oder dergleichen.

Die parenterale Verabreichung kann unter Verwendung flüssiger Dosierungsformen, wie steriler Lösungen und Suspensionen erfolgen, die für die intramuskuläre oder subcutane Injektion bestimmt sind. Derartige Dosierungsformen werden durch Lösen bzw. Suspendieren einer abgemessenen Wirkstoffmenge in einem geeigneten physiologisch unbedenklichen Verdünnungsmittel wie beispielsweise einem wäßrigen oder öligen Medium und Sterilisierung der Lösung bzw. der Suspension gegebenenfalls unter Mitverwendung von geeigneten Stabilisatoren, Emulgatoren und/oder Konservierungsmitteln und/oder Antioxidantien hergestellt.

Die orale Applikationsform ist, insbesondere unter dem Gesichtspunkt einer langen Therapiedauer, bevorzugt und stellt eine wesentliche Erleichterung in der Prävention und Therapie oben angeführte Krankheiten dar.

Die pharmazeutischen Präparate werden nach allgemein üblichen Verfahren hergestellt. Das Dosierungsschema kann vom Typ, Alter, Gewicht, Geschlecht und medizinischen Zustand des Patienten oder Risikogefährdeten abhängen.

In den folgenden Beispielen wird die Erfindung in weiteren Details beschrieben. Prozentangaben beziehen sich auf das Gewicht, wenn nicht anders angegeben.

1. Charakterisierung der Verbindungen

Beispiel 1.1

SM 255 (Decarestrictin M)

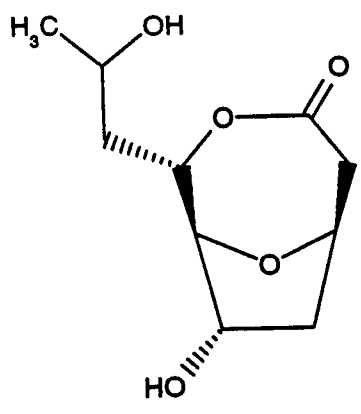

Dünnschichtchromatographie:
Kieselgel 60, $F_{254}$

a) n-Butanol/$CH_3COOH/H_2O$ = 4/1/5: $R_F$ 0.24
b) Chloroform/Methanol = 9/1: $R_F$ 0.65
c) Ethylacetat/n-Hexan = 3/1: $R_F$ 0.20

$^1$H-NMR-Spektrum (360 MHz, $D_6$-DMSO/TMS)
5.02 (OH), 4.61 (OH), 4.46 (m, 1H), 4.29 (m, 1H), 4.20 (m, 1H), 3.85 (m, 1H), 3.69 (m, 1H), 2.92 (m, 1H), 2.65 (m, 1H), 2.04 (m, 1H), 1.89 (m, 1H), 1.81 (m, 1H), 1.53 (m, 1H), 1.08 (m, 1H)
$^{13}$C-NMR-Spektrum (90.5 MHz, D6-DMSO/TMS):
171.85, 86.93, 76.37, 72.94, 71.08, 62.11, 45.14, 41.1, 40.8, 23.97
IR-Spektrum (KBr): 3370, 3240, 2960, 1755, 1730, 1250, 1160, 1080, 1000, 980, 820, 655 $cm^{-1}$
UV-Spektrum (MeOH): Endabsorption
MS: m/e = 198 ($M^+$-$H_2O$)
Drehwert $[\alpha]^{20}_D$: +22.5 (in Methanol, c=0.163)

| Elemementaranalyse: | |
|---|---|
| gef.: C 55.4<br>gef.: H 7.3<br>gef.: O 37.4 | ber.: C 55.5<br>ber.: H 7.5<br>ber.: O 37.0 |

Beispiel 1.2.

SM 240

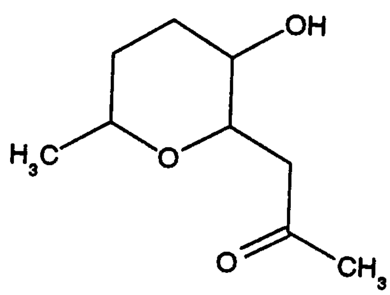

Dünnschichtchromatographie:
Kieselgel 60, $F_{254}$

a) n-Butanol/$CH_3COOH/H_2O$ = 4/1/5: $R_F$ 0.48
b) Chloroform/Methanol = 9/1: $R_F$ 0.73
c) Ethylacetat/n-Hexan = 3/1: $R_F$ 0.42

$^1$H-NMR-Spektrum (360 MHz, $CDCl_3$/TMS):
4.01 (m, 1H), 3.95 (m, 1H), 3.39 (m, 1H), 2.89 (OH), 2.76 (m, 1H), 2.69 (m, 1H), 2.21 (s, 3H), 1.87 (m, 2H), 1.73 (m, 1H), 1.72 (m, 1H), 1.58 (m, 2H), 1.22 (d, 3H, J = 7Hz)
$^{13}$C-NMR-Spektrum (90,5 MHz, $CDCl_3$ TMS):
207.87, 71.97, 69.10, 67.38, 46.16, 30.45, 23.17, 26.91, 18.14
IR-Spektrum (KBr):
3400 (OH), 2975, 2938, 1705, 1360, 1130, 1055, 1010 $cm^{-1}$
UV-Spektrum (MeOH): Endabsorption
MS: $C_9H_{16}O_3$: 172.23 e/$m^+$ = 154 ($M^+$-$H_2O$)

| Elementaranalyse: | gef.: C 62.6 %<br>H 9.1 % | ber.: C 62.8 %<br>H 9.4% |
|---|---|---|

Beispiel 1.3.

SM 140 G

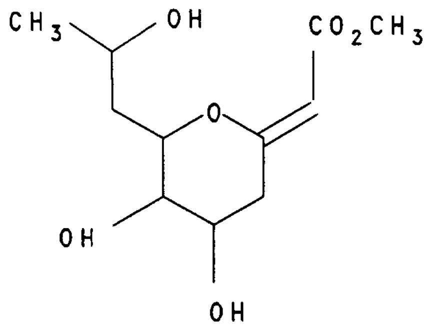

Dünnschichtchromatographie:
Kieselgel 60, $F_{254}$
Dichlormethan/Methanol = 9/1: $R_F$ 0.63
$^{1}$H-NMR-Spektrum (200 MHz, $CDCl_3$/MeOD/TMS):
5.35 (1H, s), 4.45 (1H, m), 4.20 (2H, 2m), 4.05 (1H, m), 3.65 (3H, s, $OCH_3$), 3.40 (1H, m), 3.1 (1H, psd, J = 18Hz), 1.7 (2H, 2m), 1.25 (3 Hd, J = 6 HZ)
$^{13}$C-NMR-Spektrum (50,3 $MH_2$, $CDCl_3$/MeOD/TMS):
176.2, 169.4, 92.9, 89.9, 70.4, 67.8, 64.3, 50.7, 41.3, 40.0, 23.4

Beispiel 1.4.

SM 140 H

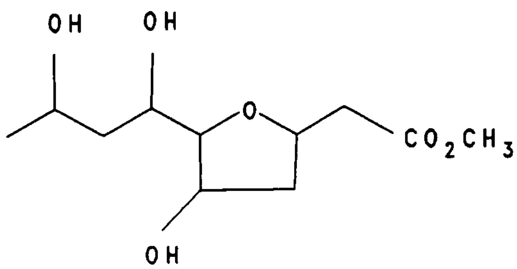

Dünnschichtchromatographie:
Kieselgel 60, $F_{254}$
Dichlormethan/Methanol = 9/1: $R_F$ 0.42
$^{1}$H-NMR-Spektrum (360 MHz, $D_6$-DMSO/TMS):
4.84 (1H, OH, d, J = 4.27), 4.30 (1H, m), 4.29 (1H, d, OH, J = 5.15 Hz), 4.13 (1H, d, OH, J = 6.54), 4.07 (1H, m, J = 2.62 Hz, J = 4.27 Hz, J = 6.17 Hz, J = 1.74 Hz), 3.78 (1H, m, J = 5.15 Hz, J = 6.24 Hz), 3.63 (1H, m, J = 3.89 Hz, J = 6.54 Hz), 3.59 (3H, s), 3.45 (1H, m, J = 3,89 Hz, J = 2,62), 2.55 (1H, m), 2,53 (1H, M), 1.79 (1H, m, J = 1.74 Hz, J = 5.34 Hz, J = -12,53), 1.58 (1H, m, J = 9,95 Hz, J = 6.17 Hz, J = -12.53 Hz), 1.36 (1H, m), 1.36 (1H, m), 1.34 (1H, m), 1.05 (3H, d, J = 6.24 Hz)
$^{13}$C-NMR-Spektrum (90.5 MHz, $D_6$-DMSO/TMS)
171.18, 90.38, 73.87, 72.13, 67.22, 62.60, 51.13, 42.86, 40.96, 39.50, 24.41

Beispiel 1.5.

SM 140 I

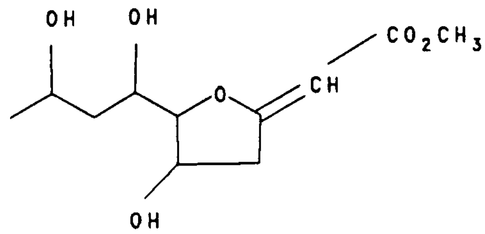

Dünnschichtchromatographie:
Kieselgel 60, $F_{254}$
Dichlormethan/Methanol = 9/1: $R_F$ 0.40
$^1$H-NMR-Spektrum (360 MHz, $D_6$-DMSO/TMS):
5.21 (1H, OH), 5.15 (1H, s), 4.71 (1H, OH), 4.34 (1H, OH), 4.28 (1H, m), 4.12 (1H, dd, J = 1,9 Hz, J = 2,6 Hz) 3.76 (1H, m), 3.76 (1H, m), 3.50 (3H, s), 3.07 (1H, dd, J = -18 Hz, J = 6,1 Hz), 2,96 (1H, dd, J = -18,1 Hz, J = 1.6 Hz), 1,41 (1H, m), 1,36 (1H, m), 1,06 (3H, d, J = 6,0 Hz)
$^{13}$C-NMR-Spektrum (90.5 MHz, $D_6$-DMSO/TMS)
177.43, 167.80, 94.57, 87.97, 69.73, 66.89, 62.46, 50.07, 42.51, 40.46, 24.33

2. Die biologische Wirksamkeit der Verbindungen als Inhibitor an der Cholesterinbiosynthese läßt sich in vitro in Leberzellkulturen nachweisen.

Monolayer von HEP-G2-Zellen in lipoproteinfreiem Nährmedium werden mit entsprechenden Konzentrationen der zu prüfenden Substanzen der allgemeinen Formel I eine Stunde vorinkubiert. Nach Zugabe der $^{14}$C-markierten Biosynthesevorstufe ($^{14}$C)Natriumacetat wird die Inkubation für 3 Stunden fortgesetzt. Danach wird ein Teil der Zellen alkalisch verseift, bei vorheriger Zugabe eines internen Standards von $^3$H-Cholesterin. Die Lipide der verseiften Zellen werden mit einem Gemisch aus Chloroform-Methanol extrahiert. Dieses Lipidgemisch wird nach Zusatz von Trägercholesterin präparativ dünnschichtchromatographisch aufgetrennt, die Cholesterinbande nach Anfärbung isoliert und die aus dem $^{14}$C-Precursor gebildete Menge $^{14}$C-Cholesterin szintigraphisch bestimmt. In einem aliquoten Teil der Zellen wurde Zellprotein bestimmt, so daß die in der Zelleinheit pro mg Zellprotein aus $^{14}$C-Präkusor gebildete Menge $^{14}$C-Cholesterins berechnet werden kann. Zum Vergleich für die Hemmwirkung eines zugesetzten Prüfpräparates dient die Kontrolle, so daß direkt die Hemmung der Cholesterinbiosynthese bei einer bestimmten molaren Konzentration des Prüfpräparates im Medium angegeben werden kann. In aliquoten Anteilen der Zellkultur wird die Intaktheit der Zellkultur und die fehlende Zellschädigung durch Präparateeinwirkung morphologisch (Lichtmikroskopie) beurteilt und biochemisch durch Bestimmung der Lactat-Dehydrogenase-Abgabe ins Inkubationsmedium gemessen.

Als Standardpräparate dienen Lovastatin ($IC_{50}$: 2,3 • $10^{-8}$ molar) und 25-Hydroxycholesterin ($IC_{50}$ 2,3 • $10^{-7}$ molar). Die Verbindung der Formeln 1.1, 1.4 und 1.5 hemmen die Biosynthese wie folgt:
Formel
1.1 - Hemmung um 69%
1.4 - Hemmung um 38 %
1.5 - Hemmung um 60 %.
(Die Angaben beziehen sich jeweils auf eine Konzentration von $10^{-8}$ mol/l).

Eine antibakterielle Wirkung kann durch übliche Hemmhoftests in vitro gezeigt werden. Die Verbindungen der Formel I zeigen besonders bei Staphylococcus aureus gute Wirkung. Die maximale Hemmkonzentration liegt im Bereich von >10 bis < 100 $\mu$g/ml.

**Patentansprüche**

**1.** Verbindungen der Formel I

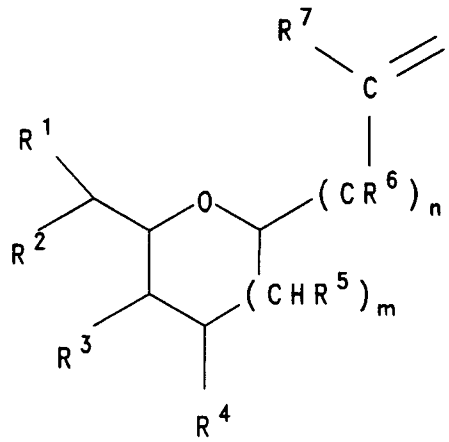

in der

| | |
|---|---|
| $R^1$ | Wasserstoff oder Hydroxyl, oder zusammen mit $R^7$ eine Sauerstoffbrücke bedeutet, |
| $R^2$ | Wasserstoff oder ein $C_2$-$C_4$-Hydroxyalkyl ist, |
| $R^3$ und $R^4$ | Wasserstoff oder Hydroxyl bedeutet, wobei mindestens ein Substituent Hydroxyl sein muß, |
| $R^5$ | Wasserstoff oder Hydroxy ist, |
| $R^6$ | Wasserstoff oder eine Doppelbindung zum Ring-C bedeutet, |
| $R^7$ | Methyl oder Methoxy und unabhängig voneinander m und n = 0 oder 1 sind. |

**2.** Verfahren zur Herstellung der Verbindungen nach Anspruch 1, dadurch gekennzeichnet, daß Penicillium sp. in einem Nährmedium kultiviert wird, bis sich die Verbindung der Formel I in der Kultur anhäuft.

**3.** Verfahren nach Anspruch 2, dadurch gekennzeichnet, daß die Stämme DSM 4209 oder DSM 4210 kultiviert werden.

**4.** Verfahren nach einem oder mehreren der oben genannten Ansprüche, dadurch gekennzeichnet, daß das Nährmedium 0,2 bis 5 % Malzextrakt, 0,02 bis 0,5 % Hefeextrakt, 0,1 bis 5 % Glucose sowie 0,005 bis 0,2 % Ammoniumsalz enthält, jeweils bezogen auf das Gewicht der gesamten Nährlösung.

**5.** Verfahren nach Anspruch 4, dadurch gekennzeichnet, daß das Nährmedium 1 bis 4 % Malzextrakt, 0,1 bis 0,4 % Hefeextrakt, 0,5 bis 2 % Glucose und 0,01 bis 0,1 % Ammoniumsalz enthält.

**6.** Verfahren nach einem oder mehreren der Ansprüche 1 bis 5, dadurch gekennzeichnet, daß die Kultivierung bei 25 bis 30 ° C erfolgt.

**7.** Verfahren nach einem oder mehreren der Ansprüche 1 bis 6, dadurch gekennzeichnet, daß die Kultivierung in dem pH-Bereich zwischen 1 und 4 erfolgt.

**8.** Verfahren zur Herstellung der Verbindungen der Formel I nach Anspruch 1 zur Anwendung in einem therapeutischen Verfahren.

**9.** Verwendung der Verbindungen nach Anspruch 1 zur Herstellung eines antibakteriellen und lipidregulatorischen Arzneimittels.

**Patentansprüche für folgenden Vertragsstaat : ES**

**1.** Verfahren zur Herstellung der Verbindungen der Formel I

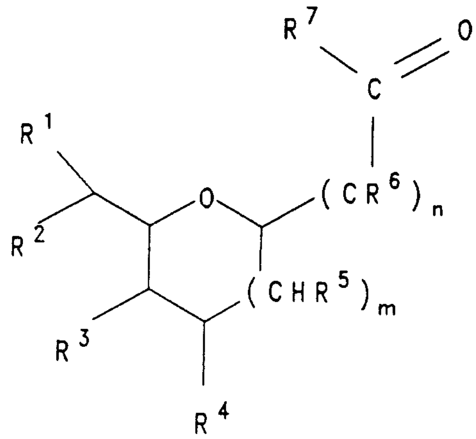

in der

| | |
|---|---|
| $R^1$ | Wasserstoff oder Hydroxyl, oder zusammen mit $R^7$ eine Sauerstoffbrücke bedeutet, |
| $R^2$ | Waserstoff oder ein $C_2$-$C_4$-Hydroxyalkyl ist, |
| $R^3$ und $R^4$ | Wasserstoff oder Hydroxyl bedeutet, wobei mindestens ein Substituent Hydroxyl sein muß, |
| $R^5$ | Wasserstoff oder Hydroxyl ist, |
| $R^6$ | Wasserstoff oder eine Doppelbindung zum Ring-C bedeutet, |
| $R^7$ | Methyl oder Methoxy und unabhängig voneinander m und n = 0 oder 1 sind, |

dadurch gekennzeichnet, daß Penicillium sp. in einem Nährmedium kultiviert wird, bis sich die VErbindung der Formel I in der Kultur anhäuft.

2. Verfahren nach Anspruch 1, dadurch gekennzeichnet, daß Penicillium sp. bei einer Temperatur von 18 bis 35 ° C, in einem pH-Bereich von 1 - 8 und über einen Zeitraum von 60 - 170 Stunden kultiviert wird.

3. Verfahren nach Anspruch 1 oder 2, dadurch gekennzeichnet, daß die Stämme DSM 4209 oder DSM 4210 kultiviert werden.

4. Verfahren nach einem oder mehreren der oben genannten Ansprüche, dadurch gekennzeichnet, daß das Nährmedium 0,2 bis 5 % Malzextrakt, 0,02 bis 0,5 % Hefeextrakt, 0,1 bis 5 % Glucose sowie 0,005 bis 0,2 % Ammoniumsalz enthält, jeweils bezogen auf das Gewicht der gesamten Nährlösung.

5. Verfahren nach Anspruch 4, dadurch gekennzeichnet, daß das Nährmedium 1 bis 4 % Malzextrakt, 0,1 bis 0,4 % Hefeextrakt, 0,5 bis 2 % Glucose und 0,01 bis 0,1 % Ammoniumsalz enthält.

6. Verfahren nach einem oder mehreren der Ansprüche 1 bis 5, dadurch gekennzeichnet, daß die Kultivierung bei 25 bis 30 ° C erfolgt.

7. Verfahren nach einem oder mehreren der Ansprüche 1 bis 6, dadurch gekennzeichnet, daß die Kultivierung in dem pH-Bereich zwischen 1 und 4 erfolgt.

8. Verfahren zur Herstellung der Verbindungen der Formel I nach Anspruch 1 zur Anwendung in einem therapeutischen Verfahren.

9. Verwendung der Verbindungen nach Anspruch 1 zur Herstellung eines antibakteriellen und lipidregulatorischen Arzneimittels.

Europäisches Patentamt

# EUROPÄISCHER RECHERCHENBERICHT

Nummer der Anmeldung

EP 92 10 1442

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-0 337 152 (HOECHST AKTIENGESELLSCHAFT) * Seite 3 - Zeile 31 * | 1,9 | C07D307/20 C07D307/32 C07D309/30 C07D309/10 C07D493/08 C12P17/04 C12P17/06 C12P17/08 A61K31/335 //(C07D493/08, 321:00,307:00), (C12P17/04, C12R1:80) (C12P17/06, C12R1:80) (C12P17/08, C12R1:80) |
| A,D | EP-A-0 333 024 (HOECHST AKTIENGESELLSCHAFT) * Seite 2 - Zeile 25 * | 1,9 | |
| A | US-A-4 952 604 (OTTO D.HENSENS ET AL.) * Spalte 1 - Zeile 62 * | 1,9 | |
| A | BULLETIN OF THE CHEMICAL SOCIETY OF JAPAN Bd. 56, Nr. 9, 1983, TOKIO Seiten 2661 - 2679; RYOJI NOYORI ET AL.: 'The Baeyer-Villiger Oxidation of 8-Oxabicyclo(3.2.1)octan-3-ones. Substituent Effects on the Regioselectivity' * Seite 2661 - Seite 2663 * | 1 | |
| | | | **RECHERCHIERTE SACHGEBIETE (Int. Cl.5)** |
| | | | C07D C12P |

Der vorliegende Recherchenbericht wurde für alle Patentansprüche erstellt

| Recherchenort | Abschlußdatum der Recherche | Prüfer |
|---|---|---|
| BERLIN | 07 MAI 1992 | KYRIAKAKOU G. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)